# NEW EUROPEAN PATENT SPECIFICATION

(11) **EP 0 137 178 B2**
(45) Date of publication and mention of the opposition decision: **10.11.1999**
(45) Mention of the grant of the patent: 23.05.1990
(21) Application number: 84109141.6
(22) Date of filing: 01.08.1984
(51) Int. Cl.: A61K 7/13

(54) **Colouring shampoo**
Waschtönungsmittel
Shampooing colorant

(30) Priority: 11.08.1983 GB 8321653
(43) Date of publication of application: 17.04.1985
(73) Proprietor: Wella Aktiengesellschaft, 64295 Darmstadt (DE)
(72) Inventor: Houiellebecq, Terry Francis, Chertsey Surrey (GB); Day, Martin Edwin, Frimley Camberley Surrey (GB); Wilkins, Anne Lilian, Twickenham Middlesex (GB)
(74) Representative: KEIL & SCHAAFHAUSEN

(56) References cited:
- DE-B- 2 154 547
- FR-A- 2 096 377
- FR-A- 2 112 489
- GB-A- 986 712
- US-A- 3 092 555
- US-A- 3 986 825
- US-A- 4 362 528
- Surfactants in Cosmetics (M. Rieger Ed.) (Dekker 1985), "Cocamido propyl betaine"
- HANDBUCH DER GESAMTEN PARFUMERIE UND KOSMETIK, 1932, Seite 517, "Henna-Shampoon"
- KOSMETOLOGIE, 1967, Seite 537

## Description

The invention relates to dye compositions useful in dyeing natural fibres, and in particular for dyeing human hair.

Direct dyes are commonly used to colour fibrous materials, for instance human hair, for a limited time, and are particularly useful when repeated changes in colour are required. However, direct dyes often impart a low colour intensity to a substrate and safety considerations may prevent the use of more concentrated compositions.

It has now been found that the colour intensity of direct nitro dyes deposited from shampoo-based compositions can be increased by the use of auxiliary surfactants, the auxiliary surfactant facilitating higher deposition of dye onto the substrate.

Accordingly, the present invention provides a dye composition for treating hair consisting of
- 0,01 to 10% by weight of a direct nitro dye,
- 5 to 20% by weight of an anionic shampoo base,
- 1 to 10% by weight of an auxiliary surfactant which is a betaine surfactant and conventional ancillary ingredients such as conditioning agents, opacifiers, pearlescent agents, sequestrants, perfumes, preservatives, glycols and water.

A suitable direct nitro dye is for example a nitrophenylene diamine. The dye will normally be present in the composition of the invention in an amount of from 0,01 to 5% by weight, preferably in an amount of from 0,01 to 3% by weight.

Anionic shampoo bases comprise a major proportion of an anionic surfactant (hereinafter the "primary surfactant") and may optionally include additional surfactants (hereinafter the "secondary surfactants") which may be anionic, non-ionic, or cationic surfactants to modify the cleaning, foaming and conditioning properties of the shampoo base.

Suitable primary surfactants for use in the compositions of the invention include sodium and ammonium lauryl sulphates, sodium and ammonium lauryl ether sulphates, sodium olefin sulphonates, substituted ammonium lauryl sulphates and substituted ammonium lauryl ether sulphates, fatty acid alkanolamide sulphosuccinates and fatty acid sarcosinates.

Suitable betaine surfactants include compounds of the formula: 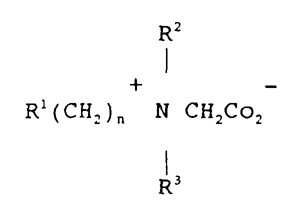 wherein:
- R¹ is a C₁₀₋₁₈ alkyl group or a C₉₋₁₇ alkyl carbonylamino group;
- R² is a C₁₋₃ alkyl group or a hydroxy C₁₋₃ alkyl group;
- R³ is a C₁₋₃ alkyl group or a hydroxy C₁₋₃ alkyl group;
   and ₙ is an integer from 1 to 5.

Suitable non-ionic secondary surfactants include fatty acid alkanolamides, polyalkoxylated fatty acid amides, polyalkoxylated sorbitan esters of long chain fatty acids, polyalkoxylated long chain alkylamine oxides and amido amine oxides of long chain fatty acids may also be used.

Preferably, the betaine surfactant is a cocamido alkyl betaine such as cocamido propyl betaine.

The pH of the compositions of the invention suitably may be from 4 to 10, preferably from 5 to 8. If necessary the pH may be adjusted using conventional agents.

The present invention also provides a process for the preparation of a dye composition, which process comprises the following main steps:
a) preparing the anionic shampoo base by admixing the primary surfactant and if required any secondary surfactant with water at ambient or slightly elevated temperature;
b) admixing the auxiliary surfactant, pre-dissolved in water if necessary, and the anionic shampoo base at ambient or slightly elevated temperature;
c) adding the direct nitro dye;
d) and finally adjusting the pH of the composition to within the range of from 4 to 10, preferably from 5 to 8.

Further toiletries additives may be added at any convenient stage in the process preferably before final pH adjustment.

The direct nitro dye may be in dry powder form or may be predispersed, suspended or dissolved in a suitable solvent, preferably a glycol, for example glycerin or propylene glycol.

The invention further provides a method for treating non-human or human hair comprising applying an effective, non-toxic amount of a composition as hereinbefore defined to the hair.

The composition of the invention will now be illustrated by way of the following Example.

### Example

| | % by weight |
|---|---|
| * Monoethanolamine lauryl ether sulphate | 8.75 |
| * Polyoxyethylene (3) lauramine oxide | 1.00 |
| Cocamido propyl betaine | 4.50 |
| * Glycerin | 4.50 |
| 2-Nitro-p-phenylene diamine | 0.40 |
| * Preservatives, perfume, etc. | qs |
| * Water | qs 100.0 |

| | |
|---|---|
| * = conventional anionic shampoo base | |

### Comparative Tests

A number of test formulations were prepared together with a corresponding number of control formulations in which the auxiliary surfactant was omitted. A number of hair tresses were prepared using light brown virgin human hair, each tress of hair (1 g) being bound with waxed twine.

### Formulation 1

| | Weight Percent |
|---|---|
| Ammonium lauryl ether sulphate | 12.0 |
| Polyoxyethylene (20) sorbitan mono-oleate | 4.0 |
| ** Cocamido propyl betaine | 4.0 |
| Glycerin | 4.5 |
| 2-Nitro-p-phenylene diamine | 0.3 |
| 4-Nitro-o-phenylene diamine | 0.3 |
| Perfume | qs |
| Preservatives | qs |
| Deionised water | qs to 100.0 |

| | |
|---|---|
| **Note** ** Indicates the auxiliary surfactant in each of the above test formulations, omitted from the control formulations. | |

### Formulation 2

| | Weight Percent |
|---|---|
| Monoethanolamine lauryl ether sulphate | 10.0 |
| Linoleic diethanolamide | 4.0 |
| ** Cocamido propyl betaine | 4.0 |
| Glycerin | 4.5 |
| 2-Nitro-p-phenylene diamine | 0.3 |
| 4-Nitro-o-phenylene diamine | 0.3 |
| Perfume | qs |
| Preservatives | qs |
| Deionised water | qs to 100.0 |

| | |
|---|---|
| **Note** ** Indicates the auxiliary surfactant in each of the above test formulations, omitted from the control formulations. | |

### Test Procedure

Each tress (1 g) was damped with water then treated with a sample (1 g) of a test or a control formulation and left for 10 minutes before rinsing with warm water and drying. The colour, shade, and intensity of tresses dyed with each test formulation was compared with that of the tresses dyed with the corresponding control formulation.

### Results

| **Formulation** | **Colour** | **Comparision with control formulation** |
|---|---|---|
| 1 | Warm, hazel brown | Brighter colour, stronger red shade |
| 2 | Golden brown | More intense |

## Claims

1. A dye composition for treating hair consisting of
- 0,01 to 10% by weight of a direct nitro dye,
- 5 to 20% by weight of an anionic shampoo base,
- 1 to 10% by weight of an auxiliary surfactant which is a betaine surfactant
and conventional ancillary ingredients such as conditioning agents, opacifiers, pearlescent agents, sequestrants, perfumes, preservatives, glycols and water.

2. A dye composition as claimed in claim 1, wherein the anionic shampoo base comprises a major proportion of an anionic surfactant.

3. A dye composition as claimed in claim 1 or 2, wherein the betaine surfactant is a compound of the formula: 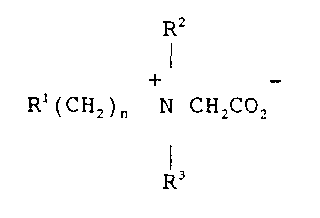 wherein:
- R¹ is a C₁₀₋₁₈ alkyl group or a C₉₋₁₇ alkyl carbonylamino group;
- R² is a C₁₋₃ alkyl group or a hydroxy C₁₋₃ alkyl group;
- R³ is a C₁₋₃ alkyl group or a hydroxy C₁₋₃ alkyl group;
and ₙ is an integer from 1 to 5.

4. A dye composition as claimed in any one of claims 1 to 3, wherein the betaine surfactant is a cocamido alkyl betaine.

5. A dye composition as claimed in any one of claims 1 to 3, wherein the betaine surfactant is a cocamido propyl betaine.

## Patentansprüche

1. Eine Farbstoffzusammensetzung für die Haarbehandlung bestehend aus
- 0,01 bis 10 Gewichtsprozent eines Nitro-Direktfarbstoffes,
- 5 bis 20 Gewichtsprozent einer anionischen Shampoo-Grundsubstanz,
- 1 bis 10 Gewichtsprozent eines zusätzlichen oberflächenaktiven Mittels, welches ein oberflächenaktives Mittel auf Betainbasis ist und
üblichen Hilfsstoffen wie Konditioniermitteln, Trübungsmitteln, Perlglanzmitteln, Maskierungsmitteln, Parfums, Konservierungsstoffen, Glykolen und Wasser.

2. Eine Farbstoffzusammensetzung nach Anspruch 1, in der die anionische Shampoo-Grundsubstanz einen größeren Anteil eines anionischen, oberflächenaktiven Mittels umfaßt.

3. Eine Farbstoffzusammensetzung nach den Ansprüchen 1 oder 2, in denen das oberflächenaktive Mittel auf Betainbasis eine Verbindung der Formel 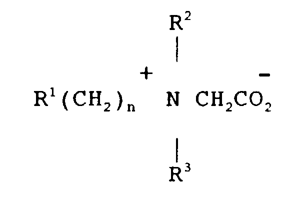 in der
- R¹ eine C₁₀₋₁₈ Alkylgruppe oder eine C₉₋₁₇ Alkylcarbonylaminogruppe;
- R² eine C₁₋₃ Alkylgruppe oder eine C₁₋₃ Hydroxyalkylgruppe;
- R³ eine C₁₋₃ Alkylgruppe oder eine C₁₋₃ Hydroxyalkylgruppe und
ₙ eine Zahl zwischen 1 und 5 ist.

4. Eine Farbstoffzusammensetzung nach den Ansprüchen 1 bis 3, in denen das oberflächenaktive Mittel auf Betainbasis ein Cocamidoalkylbetain ist.

5. Eine Farbstoffzusammensetzung gemäß den Ansprüchen 1 bis 3, worin das oberflächenaktive Mittel auf Betainbasis ein Cocamidopropylbetain ist.

## Revendications

1. Composition de colorant pour traiter des cheveux, caractérisée en ce qu'elle comprend:
- de 0,01 à 10% en poids d'un nitro-colorant direct
- de 5 à 20% en poids de la base de shampooing anionique,
- de 1 à 10% en poids d'un tensioactiv auxiliaire qui est un tensioactif de type bétaîne et
agents auxiliaires normales comme agents condionnants, agents troublants, agents nacrants, agents de masquage, parfums, agents conservateurs, glycols et eaux.

2. Composition de colorant suivant la revendication 1, caractérisée en ce que la base de shampooing anionique comprend une proportion majeure d'un tensioactif anionique.

3. Composition de colorant suivant la revendication 1 ou la revendication 2, caractérisée en ce qui le tensioactif de type bétaîne est un composé de formule: 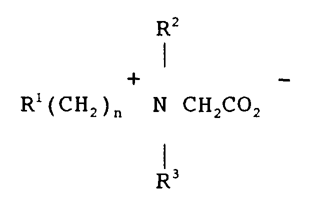 dans laquelle
- R¹ est un groupe alkyle en C₁₀₋₁₈ ou un groupe alkyle C₉₋₁₇ carbonylamino;
- R² est un groupe alkyle en C₁₋₃ ou hydroxyalkyle en C₁₋₃;
- R³ est un groupe alkyle en C₁₋₃ ou hydroxyalkyle en C₁₋₃
et ₙ est un entier de 1 à 5.

4. Composition de colorant suivant l'une quelconque des revendications 1 à 3, caractérisée en ce que le tensioactif de type bétaîne est une cocamidoalkylbétaîne.

5. Composition de colorant suivant l'une quelconque des revendications 1 à 3, caractérisée en ce que le tensioactif de type bétaîne est une cocamidopropylbétaîne.
